# EUROPEAN PATENT APPLICATION

(11) **EP 2 777 505 A1**
(43) Date of publication of application: **17.09.2014**
(21) Application number: 14158986.1
(22) Date of filing: 11.03.2014
(51) Int. Cl.: A61B 6/14, A61B 6/00, G06F 19/00

(54) **X-ray system having a user interface with swipe and log viewing features**

(30) Priority: 12.03.2013 US 201313797232
(71) Applicant: Dental Imaging Technologies Corporation, Hatfield, PA 19440 (US)
(72) Inventor: Moellmer, Jeffrey A., Tomball, Texas 77377 (US); Savolainen, Jorma K., 00740 Helsinki (FI); Anttila, Mika J., 02200 Espoo (FI)
(74) Representative: Garratt, Peter Douglas

(57) **Abstract**

An x-ray system including an x-ray source generating an x-ray stream. The x-ray system also includes a processor in communication with the x-ray source and a user interface module. The user interface module is configured to generate a graphical user interface to control the x-ray system. The graphical user interface includes a first window and a second window. The x-ray system further includes a touch screen configured to display the graphical user interface. The touch screen is configured to generate an output in response to a swipe of the touch screen. The user interface module is configured to effect navigation between the first window and the second window in response to the swipe.

## Description

### BACKGROUND

The present invention relates to x-ray imaging, including dental x-ray imaging. More particularly, some embodiments of the invention relate to an intraoral x-ray system having a user interface with swipe and log viewing features.

X-rays have been used in dentistry to image teeth and parts of the mouth for many years. In general, the process involves generating x-rays and directing the x-rays at the patient's mouth. The x-rays are attenuated differently by different parts of the mouth (e.g., bone versus tissue) and this difference in attenuation is used to create an image by using an electronic image sensor, an imaging plate, or other type of receptor.

### SUMMARY

One challenge associated with x-ray systems relates to recording the dose of x-rays given to a patient or to which a patient is exposed and retrieving information about the previously generated x-rays. Currently, the operator of the x-ray intraoral system must manually enter the generated x-ray dose (e.g., in a computer connected to the intraoral system), which is a time consuming process that takes several steps. Further, in order to retrieve a list of previously generated x-ray doses (i.e., a log), the operator must utilize a cumbersome multi-step process.

In one embodiment, the invention provides an x-ray system including an x-ray source generating an x-ray stream. The x-ray system also includes a processor in communication with the x-ray source and a user interface module. The user interface module is configured to generate a graphical user interface ("GUI") to control the x-ray system, where the graphical user interface includes a first window and a second window. The x-ray system further includes a touch screen configured to display the graphical user interface. The touch screen is configured to generate an output in response to at least one swipe of the touch screen (such as with a user's finger). The user interface module is configured to effect navigation between the first window and the second window in response to the at least one swipe.

In another embodiment, the invention provides a method for controlling an x-ray system. The system includes an x-ray source generating an x-ray stream and a processor in communication with the x-ray source and a user interface module. The system also includes a touch screen connected to the processor and/or the user interface module. The method includes generating, by the user interface module, a graphical user interface, displaying, on the touch screen, the graphical user interface, and switching between a clinical window and a manual window in response to finger swipes of the touch screen along a first axis. The method further includes displaying, on the touch screen, at least part of a x-ray procedure log in response to a finger swipe along a second axis. Optionally, the second axis can be substantially perpendicular to the first axis.

Other aspects of the invention will become apparent by consideration of the detailed description and accompanying drawings.

### BRIEF DESCRIPTION OF THE DRAWINGS

Fig. 1 is a schematic illustration of a dental x-ray system that includes an x-ray source having a computer or controller, an intraoral receptor located in a patient's mouth, and a display device located in the shoulder or base of the arm that holds the x-ray source.
Fig. 1A is a schematic illustration of a dental x-ray system including an x-ray source, an intraoral receptor located in a patient's mouth, a computer connected to the intraoral receptor, and a display positioned at an alternative location.
Fig. 2 illustrates a window or screen generated by a user interface module designed for controlling the x-ray system of Fig. 1.
Fig. 3 illustrates a window or screen generated by a user interface module designed for controlling the x-ray system of Fig. 1 in a clinical mode.
Fig. 4 illustrates a window or screen generated by a user interface module designed for controlling the x-ray system of Fig. 1 in a manual mode.
Figs. 5-7 illustrate a usage screen, an exposure counter screen, and a last x-rays screen generated by a user interface module of the dental x-ray system of Fig. 1.
Fig. 8 illustrates a process for controlling the dental x-ray system for displaying a log of the most recent x-ray procedures performed by the system, according to an embodiment of the invention.

### DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

It should also be noted that a plurality of hardware and software based devices, as well as a plurality of different structural components may be used to implement the invention. In addition, it should be understood that embodiments of the invention may include hardware, software, and electronic components or modules that, for purposes of discussion, may be illustrated and described as if the majority of the components were implemented solely in hardware. However, one of ordinary skill in the art, and based on a reading of this detailed description, would recognize that, in at least one embodiment, the electronic based aspects of the invention may be implemented in software (e.g., stored on non-transitory computer-readable medium) executable by one or more processors. As such, it should be noted that a plurality of hardware and software based devices, as well as a plurality of different structural components may be utilized to implement the invention. Furthermore, and as described in subsequent paragraphs, the specific mechanical configurations illustrated in the drawings are intended to exemplify embodiments of the invention and that other alternative configurations are possible. For example, "controllers" described in the specification can include standard processing components, one or more microprocessors, controllers, or other programmable devices, one or more computer-readable medium modules, one or more input/output interfaces, and various connections (e.g., a system bus) connecting the components.

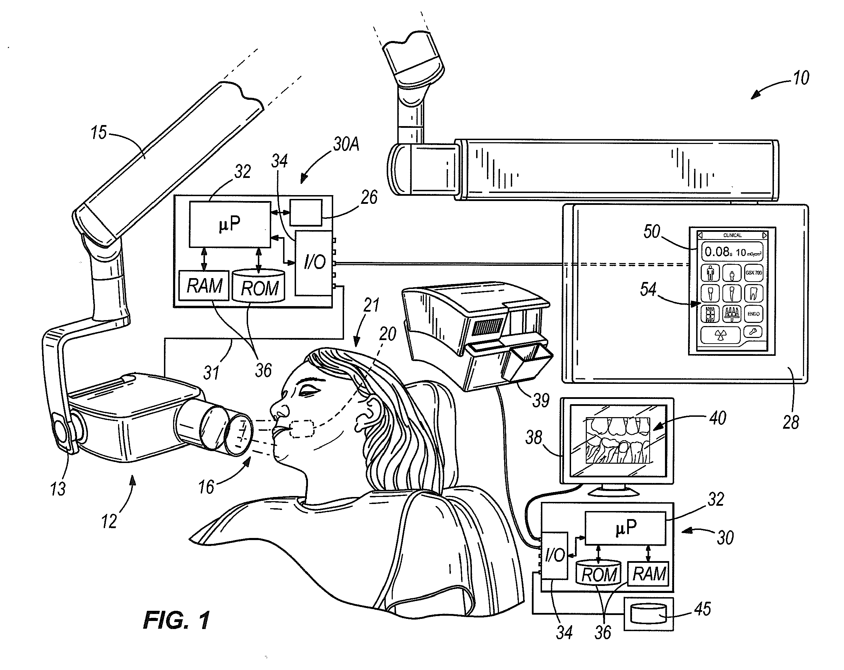

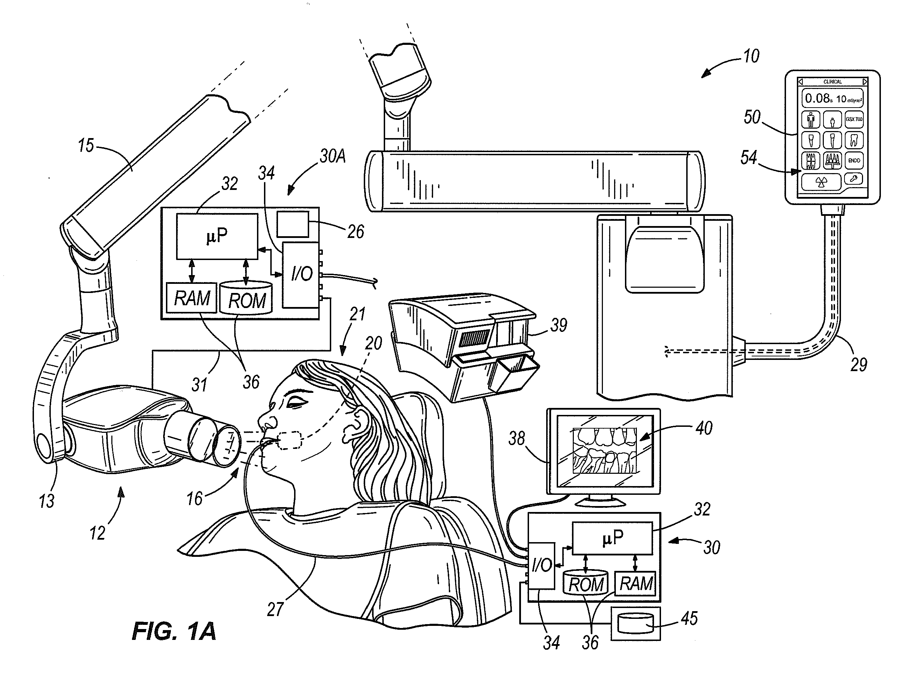

Fig. 1 illustrates an intraoral dental x-ray system 10. The system includes an x-ray source 12. In the illustrated embodiment, the source 12 is located on an end 13 of a mechanical arm 15. When activated, the x-ray source 12 generates an x-ray stream 16 that has a generally circular cross-section. (Although x-rays are generally invisible, a representation of a stream is illustrated to facilitate understanding of the invention.) In some applications, a collimator (not shown) is used to reduce the size of the stream and generate a smaller x-ray stream having a rectangular cross-section. A collimator may be used with a mechanical positioning device to help align the x-ray stream with an x-ray receptor. As shown in Fig. 1, the x-ray source 12 is positioned (e.g., by an operator (not shown)) so that the x-ray stream 16 is directed to an intraoral receptor 20. The intraoral receptor 20 is shown located in the mouth of a patient 21. The receptor 20 can include a digital detector, a sensor, a film plate, or an imaging plate (e.g., phosphorescent plate or other type of imaging plate). For example, the receptor 20 could include self-contained memory and be read in a reader after an imaging procedure by being physically moved from the patient's mouth to the reader by an operator, who can be, for example, a technician or dentist.

In Fig. 1A, the intraoral receptor 20 is a sensor and a wire, cable, or similar connector 27 connects the receptor 20 to a computer 30. The connection between the receptor 20 and the computer 30 can alternatively be a wireless connection, a fiber-optic connection, or other connection suitable for transmitting data between the devices.

Referring back to Fig. 1, the x-ray source 12 includes a computer 30A that is operable to monitor and control the operation of the x-ray system 10. In the illustrated embodiment, the computer 30A is shown outside of the housing of the x-ray source 12, and connected to the x-ray source 12 through a connection 31. However, it should be understood that the computer 30A could be located within the housing of the x-ray source 12. A touch screen 50 is electrically connected to the computer 30A. In the illustrated embodiment, the touch screen 50 is positioned in a housing 28 located at the base or shoulder of the arm 15. For illustrative purposes, in the embodiment shown in Fig. 1, the connection (i.e., a wire) between the computer 30A and the touch screen 50 is illustrated as being outside of the arm 15. However, it is to be understood that a wired connection between the computer 30A and the touch screen 50 could be accomplished by routing a wire from the x-ray source 12 to the computer 30A through all or part of the arm 15.

To simplify the description provided herein, the components of the computers 30 and 30A are given the same reference numbers. Each of computers 30 and 30A includes at least a microprocessor and can optionally also include other components for example programmable controller, or similar electronic device 32, an input/output interface 34, and memory 36 (e.g., RAM and ROM). The computer 30A further includes a user interface module 26. The user interface module 26 is shown schematically in Fig. 1. It should be understood that the user interface module 26 could include software (stored in memory, e.g., in ROM and/or RAM) and hardware (e.g., a graphics processor). In some embodiments, the input/output interface 34 includes a USB connection and the cable 27 is a USB cable. The x-ray source 12 can also be connected to the computer 30 (in addition to or instead of the computer 30A) via an electrical or other communication connection.

Fig. 1A illustrates an alternative embodiment of the intraoral dental x-ray system 10. The x-ray system 10 shown in Fig. 1A includes many of the same elements as the system shown in Fig. 1. In Fig. 1A, a touch screen 50 is electrically connected to the computer 30A through an extension 29 connected to the shoulder or base of the arm 15.

The operation of the system 10 will be described in reference to the system illustrated in Fig. 1A. In the embodiment illustrated in Fig. 1A, image data is captured by the receptor 20, the data is processed by the computer 30, and the processed data is sent to a display 38 and viewed as image 40. (Image 40 is drawn more distinctly than an x-ray image would typically appear.) It should be understood, however, that the receptor 20 could be configured to carry out all or a portion of the image processing carried out by the computer 30. In other words, imaging processing could be distributed between the receptor 20 and computer 30. Processing hardware could be located in the body of the receptor 20 or in a cable for the receptor 20.

In the case of a system using imaging plates, as illustrated in Fig. 1, the exposed plate is inserted into a plate reader 39 (commonly referred to as a computed radiography ("CR") reader), which reads the exposed areas of the plate and converts them into a digital image 40 which is sent to computer 30.

In the system shown in Fig. 1 and 1A, the computer 30A controls generating the x-ray stream 16. The computer 30 may also control the image capture or triggering of the receptor 20. As noted, the processor 32 of the computer 30A is communicatively connected to the x-ray source 12 and the processor 32 of the computer 30 is connected to the receptor 20. Typically, capture of an image 40 by an electronic receptor 20 is triggered when the receptor 20 detects an incoming x-ray signal. Thus, it is possible to generate a burst of x-ray radiation and be assured that an image will be captured by the receptor 20 during the relatively short period of x-ray exposure. The processor 32 of computer 30 also sends information to a database 45 to maintain a log of exposures (described in more detail below). The database 45 can be an internal database (i.e., stored in the memory 36) or external to the computer 30.

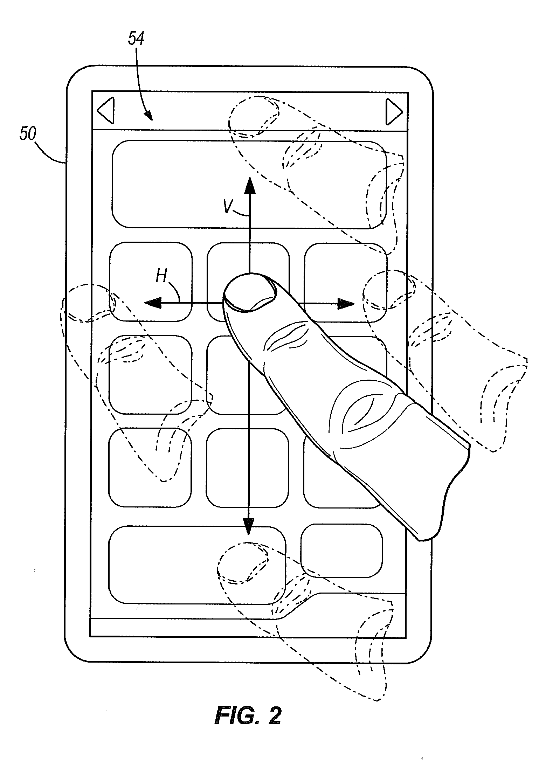

With continued reference to Fig. 1, the touch screen 50 is connected to the computer 30A via a wired (e.g., electrical) connection, a wireless connection, a fiber-optic connection, or any other suitable connection. The screens or windows, and other elements of a graphical user interface 54 (e.g., icons, menus, scroll bars, buttons, and boxes) are generated by the user interface module 26 and displayed on the touch screen 50 to control the x-ray system 10. Fig. 2 illustrates the touch screen 50 in a greater detail. The touch screen 50 may be a capacitive touch screen and a user or operator may input information to the computer 30A by touching elements of the graphical user interface 54 and by motions (e.g., swiping across the touch screen 50.) In Fig. 2, a horizontal axis, H, and a vertical axis, V, are shown and the touch screen 50 is configured to be responsive to swipes along each axis and swipes along the horizontal axis H are used to navigate to between windows while swipes along the vertical axis V are used to navigate to other features such as logs. It is, of course, possible that the touch screen 50 could be configured in a reverse fashion, with vertical swipes being used to navigate between windows and horizontal swipes used to navigate to features. Navigation from one screen to another and to, between, and in other GUI elements is explained in greater detail below. The touch screen 50 outputs a user interface signal to the processor 32 of the computer 30A in response to user touches and swipes. The processor 32 of computer 30A processes the user interface signal and provides information to the interface module 26. The interface module 26 processes the information regarding the user input and provides an output. The processor 32 provides the output of the interface module 26 to the touch screen 50 to modify the graphical elements displayed, including the windows, icons, etc. Alternatively, or in addition, the touch screen 50 can be configured to operate with only horizontal swipes or only vertical swipes so that, for example, one or more control windows appear first, followed by one or more log windows in response to repeated swipes. In addition, the sequence of windows appearing in response to multiple swipes can repeat itself, thus forming a looped sequence of windows.

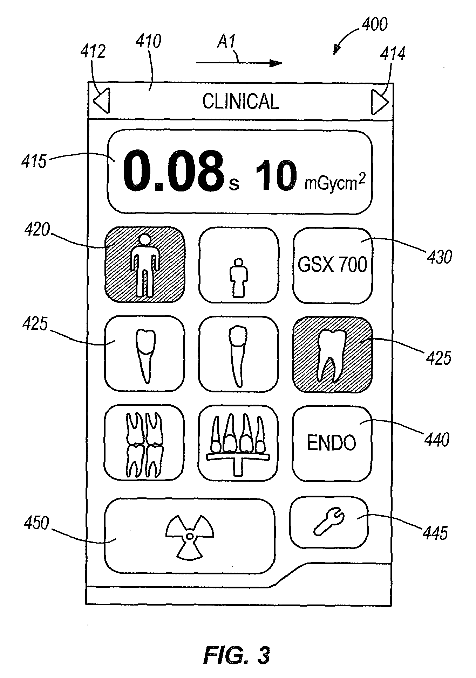

Fig. 3 illustrates a first screen 400 of the graphical user interface 54. In certain embodiments, the screen 400 is a home screen (i.e., the first screen presented to a user following boot-up). The first screen 400 includes elements (buttons and scroll bars) for operating the system 10 in a clinical mode (also known as an anatomical mode). Thus, the first screen 400 may be referred to as a clinical mode window. The first screen 400 includes elements to display information and to accept operator selections or input regarding the generated x-ray stream 16, the specific type of patient 21, the anatomy to be captured by the system 10, the imaging modality, etc.

The touch screen 50 can also display other screens or windows corresponding to other operational modes of the x-ray system 10. It should be noted that within the art of user interface design, the terms "screen," "window," and "page" are often used loosely and sometimes interchangeably, in part because users do not often draw distinctions between the terms when operating a device through a GUI. Generally, a "screen" encompasses all elements that appear on a display (hardware) at a particular moment. A "window" is an enclosed, rectangular area on a screen, often with a title bar. Different windows are used to run different programs or display different data. A "page" is a grouping of content between logical breaking points and is analogous to a hard copy page in a paper document.

The first screen or clinical mode window 400 includes a scroll bar 410 with left and right arrows 412 and 414. Although not included in the illustrated embodiment, the scroll bar 410 may include a slider. The scroll bar 410 may be referred to generically as a swipe feature. The left arrow 412 is positioned on the left end of the window 400 and the right arrow is positioned on the right end of the screen, opposite the left arrow. Scroll bar 410 is configured to allow a user to navigate between or switch to a different screen or operational mode window. In particular, the scroll bar 410 is responsive to a swipe by the operator, which involves moving a finger along the scroll bar 410 in the direction of one of the arrows 412 or 414. For example, a swipe to the right (in the direction shown by arrow A1) causes a different window to be displayed or appear on the touch screen 50. Alternatively, a user may select one of the pointers 412 or 414 to navigate between screens, windows, or windows. When a user swipes the scroll bar 410 or selects one of the pointers 412 or 414, the input from the user is provided to the computer 30A and the user interface module 26 interacts with the processor 32 to display the desired screen.

It is to be noted that, although the above description refers to navigation between screens using scroll bars, the user interface need not be limited to the use of scroll bars. It can also allow the user to swipe on other parts of the screen to perform the navigation, as illustrated in Fig. 2.

The first screen 400 also includes an x-ray time and dosage box 415. The box 415 displays information about the exposure duration (e.g., in seconds) of the x-ray stream 16 generated by the x-ray source 12 and the dose (e.g., in micro Grays square centimeters-mGycm2) of x-ray being delivered. In addition to the time and dosage box 415, the first screen 400 includes icons that are related to different functions of the x-ray system. These icons include a patient information icon 420 that represents specific information about the type of the patient 21. (i.e., adult, child, etc.); patient anatomy icons 425 that represent information about the specific type of anatomic structure that is imaged by the system 10; a system information icon 430 that represents information about the specific x-ray system 10; an "ENDO" icon 440 that provides a predefined exposure set of settings for a specific x-ray imaging projection "endodontic" image and is used in conjunction with icons 425; a settings or tools icon 445 that allows the user to adjust specific setting of the x-ray source 12; and an exposure start button or icon 450 that initiates the x-ray exposure. Typically, the illustrated exposure start button or icon 450 is used in conjunction with the extension 29 illustrated in Fig. 1A. Preferably, the extension 29 is long enough for the operator and the patient to be in different rooms or separated by x-ray shielding material, or for the operator to be sufficiently far away from the x-ray source to avoid radiation exposure. Alternatively, or in addition, a separate, remote, exposure button can be used.

In addition to navigation between screens, selections made by an operator (e.g., by touching the touch screen 50 at the point where an icon appears) result in information being input to the computer 30A. In the embodiment shown, selections are indicated by an icon being highlighted. Highlighted icons represent specific user selections related to the operating parameters of the x-ray system 10. For instance, in the example illustrated in Fig. 3, the user has selected an adult patient (i.e., icon with the image of a larger human as opposed to the icon with the smaller human, which represents a child) and wants to obtain the specifically selected dental image (i.e., highlighted in darker color) on the screen 400.

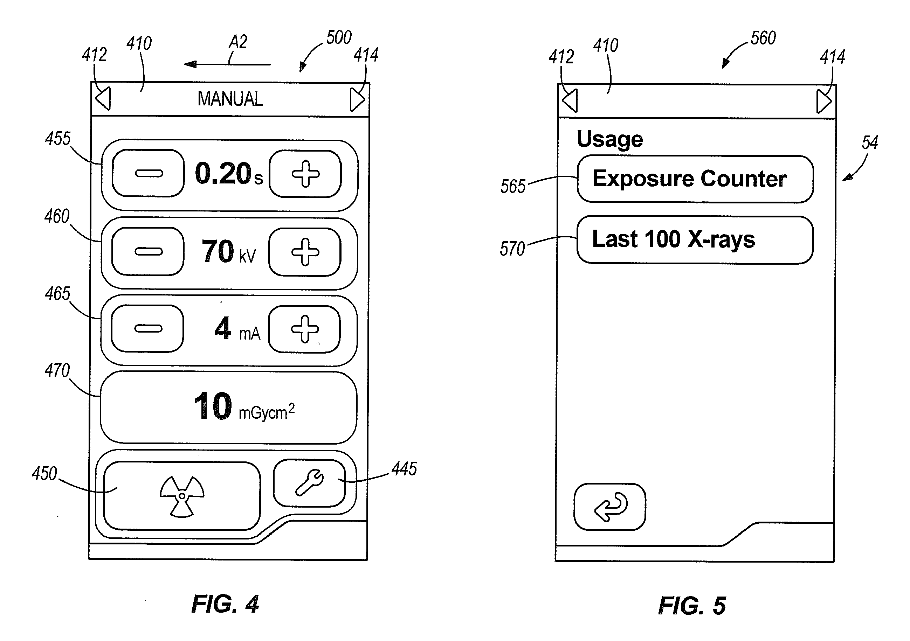

Fig. 4 illustrates a second screen 500 that includes elements for operating the system 10 in a manual mode. Other modes of operation of the x-ray system 10 include a service mode, settings modes (e.g., language, brightness, volume, etc.), and a network mode. The user interface module 26 generates additional screens corresponding to each mode. When an appropriate swipe from a user on the touch screen 50 is detected, navigation from the screen 400 to the screen 500 is accomplished. Like the first screen 400, the second screen 500 includes the scroll bar 410 with arrows 412 and 414. Thus, it is possible to navigate from the second screen 500 back to the first screen 400 and, since each of the screens 400 and 500 is associated with an operational mode, switching screens allows a user to switch operational modes. For example, a swipe to the left (in the direction shown by arrow A2) causes the touch screen 50 to switch from the second screen 500 back to the first screen 400. However, use of the scroll bar is optional; the user interface can be configured to allow the user to swipe on other parts of the screen.

The second screen 500 includes box 455, which is an exposure duration box that allows a user to modify the duration of the x-ray output or stream 16 generated by the x-ray source 12. When the user wants to decrease the x-ray exposure duration, the user physically touches the minus sign of the exposure duration box 455. Likewise, an increase in the exposure can be effected by touching the plus sign. Box 460 is a power adjustment box that allows a user to modify the voltage applied to the x-ray generator (not shown) in the x-ray source 12. Box 465 is current adjustment box that allows a user to modify the current supplied to the x-ray generator in the x-ray source 12. Adjustment of the voltage and current adjusts the intensity of the x-ray stream 16. Box 470 is an x-ray dose display section that displays the dose that will be delivered by the x-ray stream 16. The second screen 500 also includes the tools icon 445 for accessing a tools window. The exposure start button or icon 450 (also shown in the second screen 500) starts an x-ray exposure or procedure. In other embodiments, the manual mode screen 500 can include different GUI elements.

A user of the x-ray system 10 operates the graphical user interface 54 as follows. A user activates or selects an element of the graphical user interface 54 with his or her finger. The touch screen 50 provides positional information regarding the user's fingers in a manner that is similar to how the position of a cursor is tracked. In one embodiment, a "mouse click" is effected when a user taps an icon or active part of the GUI 54. When a user selects an element (without tapping) and moves his or her finger across the touch screen 50, a drag operation is performed. A drag operation moves an object on the display in accordance with movement of the user's finger. Additional functions can also be performed on the touch screen 50 (e.g., a vertical swipe, zooming in and out, pinching, etc.).

The described functionality of the touch screen 50 and the graphical user interface 54 allow a user to easily switch between the different modes of operation of the x-ray system 10. For example, when the user is in clinical mode and wants to adjust the parameters related to the x-ray stream 16, the user uses the scroll bar 410 to "move" from screen 400 to screen 500 and the corresponding manual mode.

In one embodiment, the user swipes the scroll bar 410 to the left (or a first direction) in order to display the manual mode window 500. Once the manual mode window 500 appears, the user can easily go back to the clinical mode window 400 by swiping the scroll bar 410 to the right (or a second direction opposite the first). This swipe configuration of the user interface 54 allows a user to easily switch back and forth between clinical and manual modes and to adjust the operating parameters of the x-ray source 12

It should be noted that although Figs. 3 and 4 illustrate a graphical user interface 54 configured for horizontal swiping, the graphical user interface 54 and user interface module 26 could be configured so that vertical swipes or diagonal swipes caused different screens to be displayed on the touch screen 50. In addition, the system 10 is configured so that multiple swipes of the scroll bar 410 can used to navigate through multiple windows (in sequence) to a desired window.

In addition to providing mechanisms to switch operational modes and adjust specific operational parameters, the graphical user interface 54 is configured to provide information regarding usage of the system 10 in the form of one or more logs and counters. In the embodiment described herein, the system 10 provides an exposure counter that 1) tracks or counts the number of x-ray procedures performed by the system 10 and 2) the total exposure duration. The system 10 also includes a log of x-ray procedures (or "x-rays") that includes details about a predetermined number of procedures.

During the operation of the x-ray system 10, every time that the x-ray source 12 generates an x-ray stream 16 to expose a patient 21, the processor 32 of the computer 30A retrieves information about the specific x-ray signal (e.g., date, time, exposure duration, power, current, etc.). The processor 32 then automatically stores that information in a database or in the memory 36 of the computer 30A every time an x-ray procedure is performed. Alternatively, this information can be stored in a database associated with the computer 30 or in the memory 36 of the computer 30. Further, by using the software stored in the memory 36, the processor 32 of the computer 30A systematically calculates various outputs related to the x-ray distribution of the system 10. For example, the processor 32 calculates the total number of x-rays taken by the system 10 and the total exposure duration. In one embodiment, the processor 32 creates reports with that information and stores the reports in a database or in the memory 36 of the computer 30A.

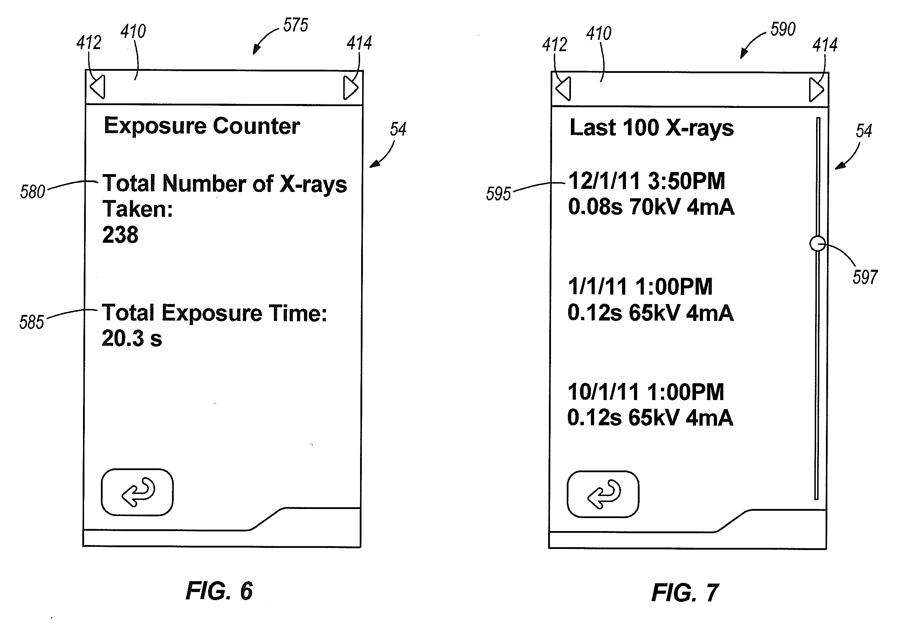

When the graphical user interface 54 displays a window in clinical or manual mode, the user utilizes the touch screen functionality of the graphical user interface 54 to switch to a screen that shows the overall usage of the system 10 in the form of one or more logs and counters. Alternatively, a user can touch or click on an icon that opens a new screen that shows the overall usage of the system 10. Figs. 5-7 represent an illustration of the above-described process. Fig. 5 shows a usage screen 560 that includes an "Exposure Counter" icon 565 and "Last 100 x-rays" icon 570. Fig. 6 illustrates an "Exposure Counter" screen 575 and Fig. 7 shows a "Last 100 x-rays" screen 590. When the graphical user interface 54 is in clinical or manual mode, a user can display the screen 560 by swiping his or her finger on the scroll bar 410 described above.

In other embodiments, rather than use an additional screen (i.e., screen 560) to access the exposure counter and procedures log, the user interface 54 is configured so that the user can directly display the "Exposure Counter" screen 575 and the "Last 100 x-rays" screen 590 from the clinical or manual mode screens. For example, when the user interface 54 displays a window in clinical or manual mode, the user can use a vertical swipe across the touch screen 50 to navigate to the "Last 100 x-rays" screen 590 directly from the clinical mode screen 400 or the manual mode screen 500. After the user's swipe, the user interface module 26 processes the user input and provides an output to the processor 32. The processor 32 generates an output to the touch screen 50 to effect navigation from the clinical mode screen 400 or the manual mode screen 500 to the "Last 100 x-rays" screen 590.

As shown in Fig. 6, the "Exposure Counter" screen 575 includes a section 580 showing the total number of x-rays taken by the system 10 and a section 585 showing the total exposure duration. In one embodiment, the number of x-rays and the exposure duration represent the parameters of the entire system 10 (i.e., information for all patients). In another embodiment, the number of x-rays and the exposure duration represent specific parameters for an individual patient 21. Computer 30A can, for example, retrieve individual patient exposure information from computer 30 through a communication connection such as an Ethernet link. As shown in Fig. 7, the "Last 100 x-rays" screen 590 includes a log with exposure information about a predetermined number of the most recent x-rays generated by the system 10. In the illustrated embodiment, the log includes the last one hundred x-rays or x-ray procedures. In other embodiments, the user can adjust the setting of the system 10 to show a log with different number of x-rays (e.g., 50, 150, 200, etc.).

The exposure information about each individual x-ray stream 16 displayed in the log is included in an exposure information section 595. Each exposure information section 595 includes the following log data: date and time of administration, exact exposure duration, x-ray dose (optionally), voltage and current of the x-ray generator. In one embodiment, the user can customize the exposure information to include more or less parameters. Initially, the screen only displays a certain number of entries or exposure information sections 595 (e.g., three in the illustrated embodiment). The user uses a vertical scroll bar 597 to scroll up and down the screen in order to view the rest of the entries in the log. In an individual x-ray session, an operator often performs multiple x-ray exposures on a patient 21. The available log feature of the system 10 allows the operator to perform the planned exposure and then quickly swipe the screen 50 to bring in the x-ray log screen 590. The displayed log includes all the exposures performed by the operator and the operator can quickly retrieve data about the number of exposures and the total x-ray dose administered to the patient 21.

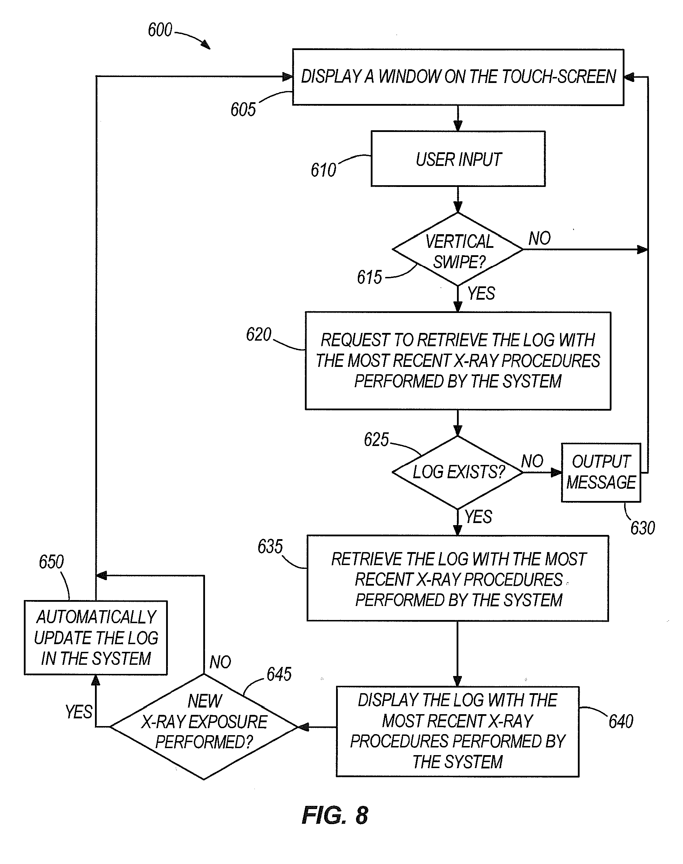

A process 600 for controlling the x-ray system 10 and for displaying a log of the most recent x-ray procedures performed by the system 10 is illustrated in Fig. 8. In some embodiments, the process 600 is executed by the processor 32 of the computer 30A. The process 600 begins with displaying a window (step 605) of the graphical user interface 54 on the touch screen 50. For example, the first window 400 (or clinical mode screen) is displayed. The processor 32 then waits for an input from the user (step 610). In other words, the processor 32 waits to receive information output by the touch screen 50 in response to a finger swipe or a touch on the screen. If the output signal from the touch screen 50 is indicative of a vertical swipe (for example, along the vertical axis V) (step 615), the user interface module 26 interprets the signal as a request for the log with the most recent x-ray procedures performed by the system (at step 620). If the swipe is not a vertical swipe (step 615), the process returns to step 605. Next, if the swipe is a vertical swipe, the processor 32 determines whether a log with the most recent x-ray procedures exists (at step 625). If no log exists, an appropriate message is output (step 630) and the process returns to step 605.

If a log exists, the processor 32 retrieves the log with the most recent x-ray procedures performed by the system 10 (at step 635). Specifically, the processor 32 accesses the memory 36 or the database 45 and retrieves the log that is stored there. Then, the user interface module 26 displays the log screen 590 with the log (at step 640). The processor 32 tracks whether a new x-ray procedure is performed (step 645) and automatically updates the log with information about the new x-ray exposure every time that a new x-ray exposure or procedure is performed (at step 650).

The steps of the process 600 have been illustrated and described in a particular manner. However, various steps described herein with respect to the process 600 are capable of being executed simultaneously, in parallel, or in an order that differs from the manner of execution illustrated in Fig. 8.

Thus, the invention provides, among other things, an intraoral x-ray system having a user interface with swipe and log viewing features. Various features and advantages of the invention are set forth in the following claims.

## Claims

1. An x-ray system comprising:
an x-ray source generating an x-ray stream;
a processor in communication with the x-ray source and a user interface module, the user interface module configured to generate a graphical user interface to control the x-ray system, the graphical user interface having a first window and a second window; and
a touch screen configured to display the graphical user interface, the touch screen configured to generate an output in response to at least one swipe of the touch screen, the user interface module configured to effect navigation between the first window and the second window in response to the at least one swipe.

2. The system of claim 1, wherein the first window comprises a clinical window and the second window comprises a manual mode window which preferably includes a swipe feature.

3. The system of claim 1 or claim 2, further comprising a memory configured to store a log of x-ray procedures performed with the x-ray system, wherein the user interface module is configured to generate a window that displays at least a part of the log.

4. The system of claim 3, wherein the user interface module effects navigation between the first window and the second window in response to a swipe of the touch screen in a first direction and effects navigation from either of the first and second windows to the log of x-ray procedures in response to a swipe of the touch screen in a second direction, the second direction substantially perpendicular to the first direction.

5. The system of claim 3 or claim 4, wherein in the memory is further configured to store a database, and wherein the computer is configured to create the log in the database.

6. The system of any one of claim 3 to claim 5, whether the processor is configured to determine whether a log exists in the database in response to the at least one swipe of the touch screen.

7. The system of any one of claim 3 to claim 6, wherein the log includes a date of x-ray administration, time of x-ray administration, exact exposure duration, x-ray dose, power and current of x-ray generator.

8. The system of any one of the preceding claims, wherein the graphical user interface module is configured to generate an exposure counter screen that provides information about a number of x-ray procedures performed by the system and the total exposure duration.

9. The system of any one of the preceding claims, further comprising a receptor.

10. The system of claim 9, wherein the receptor comprises a digital detector, a sensor, film, or an imaging plate.

11. A method for controlling an x-ray system, the system including an x-ray source generating an x-ray stream and, a processor in communication with the x-ray source and a user interface module and a touch screen connected to the computer, the method comprising:
generating, by the user interface module, a graphical user interface;
displaying, on the touch screen, the graphical user interface;
switching between a clinical window and a manual window in response to a swipe of the touch screen along a first axis which is preferably vertical;
displaying, on the touch screen, at least part of a x-ray procedure log in response to a swipe along a second axis which is preferably horizontal.

12. The method of claim 11, wherein the x-ray procedure log includes a list with most recent x-ray procedures performed by the x-ray system.

13. The method of claim 11 or claim 12, further comprising determining whether the x-ray procedure log exists in a memory of the x-ray system.

14. The method of any one of claim 11 to claim 13, further comprising determining whether a new x-ray procedure is performed by the x-ray system.

15. The method of any one of claim 11 to claim 14, further comprising updating the x-ray procedure log with information about the new x-ray procedure is performed by the x-ray system.
